# NEW EUROPEAN PATENT SPECIFICATION

(11) **EP 3 170 880 B2**
(45) Date of publication and mention of the opposition decision: **28.10.2026**
(45) Mention of the grant of the patent: 29.04.2020
(21) Application number: 16204715.3
(22) Date of filing: 27.10.2003
(51) Int. Cl.: C09K 5/04, F25B 1/00, C07C 19/08, C07C 21/18, C08J 9/14, C11D 7/50, C23G 5/028, A61P 1/00, C11D 7/28

(54) **USE OF COMPOSITIONS COMPRISING HFO-1234ZE OR HFO-1234YF AS REFRIGERANT COMPOSITION**
ZUSAMMENSETZUNGEN MIT FLUORSUBSTITUIERTEM OLEFIN
COMPOSITIONS CONTENANT DES OLÉFINES SUBSTITUÉES DE FLUORINE

(30) Priority: 25.10.2002 US 421263 P; 25.10.2002 US 421435 P
(43) Date of publication of application: 24.05.2017
(62) Document number(s) of the earlier application(s) in accordance with Art. 76 EPC: 09177661.7 / 2 163 591; 03777896.6 / 1 563 032
(73) Proprietor: Solstice Advanced Materials US, Inc., Wilmington, DE 19808 (US)
(72) Inventor: Singh, Rajiv, R, Getzville, NY New York 14068 (US); Pham, Hang, T, Amherst, NY New York 14228 (US); Wilson, David, P, East Amherst, NY New York 14051 (US); Thomas, Raymond, H, Pendleton, NY New York 14094 (US)
(74) Representative: Crooks, Elizabeth Caroline

(56) References cited:
- WO-A1-98/05732
- US-A- 6 054 064
- DATABASE WPI Week 199221, Derwent World Patents Index; AN 1992-172539, XP002343594
- DATABASE WPI Week 199737, Derwent World Patents Index; AN 1997-400990, XP002660418

## Description

### FIELD OF THE INVENTION

This invention relates to the use as a refrigerant composition, of a composition comprising 1,3,3,3-tetrafluoropropene (HFO-1234ze) or 2,3,3,3-tetrafluoropropene (HFO-1234yf), and a lubricant selected from polyol esters.

### BACKGROUND OF THE INVENTION

Fluorocarbon based fluids have found widespread use in many commercial and industrial applications. For example, fluorocarbon based fluids are frequently used as a working fluid in systems such as air conditioning, heat pump and refrigeration applications. The vapor compression cycle is one of the most commonly used type methods to accomplish cooling or heating in a refrigeration system. The vapor compression cycle usually involves the phase change of the refrigerant from the liquid to the vapor phase through heat absorption at a relatively low pressure and then from the vapor to the liquid phase through heat removal at a relatively low pressure and temperature, compressing the vapor to a relatively elevated pressure, condensing the vapor to the liquid phase through heat removal at this relatively elevated pressure and temperature, and then reducing the pressure to start the cycle over again.

While the primary purpose of refrigeration is to remove heat from an object or other fluid at a relatively low temperature, the primary purpose of a heat pump is to add heat at a higher temperature relative to the environment.

Certain fluorocarbons have been a preferred component in many heat exchange fluids, such as refrigerants, for many years in many applications. For example, fluoroalkanes, such as chlorofluoromethane and chlorofluoroethane derivatives, have gained widespread use as refrigerants in applications including air conditioning and heat pump applications owing to their unique combination of chemical and physical properties. Many of the refrigerants commonly utilized in vapor compression systems are either single component fluids or azeotropic mixtures.

Concern has increased in recent years about potential damage to the earth's atmosphere and climate, and certain chlorine-based compounds have been identified as particularly problematic in this regard. The use of chlorine-containing compositions (such as chlorofluorocarbons (CFC's), hydrochlorofluorocarbons (HCF's) and the like) as refrigerants in air-conditioning and refrigeration systems has become disfavored because of the ozone-depleting properties associated with many of such compounds. There has thus been an increasing need for new fluorocarbon and hydrofluorocarbon compounds and compositions that offer alternatives for refrigeration and heat pump applications. For example, it has become desirable to retrofit chlorine-containing refrigeration systems by replacing chlorine-containing refrigerants with non-chlorine-containing refrigerant compounds that will not deplete the ozone layer, such as hydrofluorocarbons (HFC's).

It is generally considered important, however, that any potential substitute refrigerant must also possess those properties present in many of the most widely used fluids, such as excellent heat transfer properties, chemical stability, low- or no-toxicity, non-flammability and lubricant compatibility, among others.

Applicants have come to appreciate that lubricant compatibility is of particular importance in many of applications. More particularly, it is highly desirable for refrigeration fluids to be compatible with the lubricant utilized in the compressor unit, used in most refrigeration systems. Unfortunately, many non-chlorine-containing refrigeration fluids, including HFC's, are relatively insoluble and/or immiscible in the types of lubricants used traditionally with CFC's and HFC's, including, for example, mineral oils, alkylbenzenes or poly(alpha-olefins). In order for a refrigeration fluid-lubricant combination to work at a desirable level of efficiency within a compression refrigeration, air-conditioning and/or heat pump system, the lubricant should be sufficiently soluble in the refrigeration liquid over a wide range of operating temperatures. Such solubility lowers the viscosity of the lubricant and allows it to flow more easily throughout the system. In the absence of such solubility, lubricants tend to become lodged in the coils of the evaporator of the refrigeration, air-conditioning or heat pump system, as well as other parts of the system, and thus reduce the system efficiency.

With regard to efficiency in use, it is important to note that a loss in refrigerant thermodynamic performance or energy efficiency may have secondary environmental impacts through increased fossil fuel usage arising from an increased demand for electrical energy.

Furthermore, it is generally considered desirable for CFC refrigerant substitutes to be effective without major engineering changes to conventional vapor compression technology currently used with CFC refrigerants.

Flammability is another important property for many applications. That is, it is considered either important or essential in many applications, including particularly in heat transfer applications, to use compositions which are non-flammable. Thus, it is frequently beneficial to use in such compositions compounds which are nonflammable. As used herein, the term "nonflammable" refers to compounds or compositions which are determined to be nonflammable as determined in accordance with ASTM standard E-681, dated 2002. Unfortunately, many HFC's which might otherwise be desirable for use in refrigerant compositions are not nonflammable. For example, the fluoroalkane difluoroethane (HFC-152a) and the fluoroalkene 1,1,1-trifluoropropene (HFO-1243zf) are each flammable and therefore not viable for use in many applications.

Higher fluoroalkenes, that is fluorine-substituted alkenes having at least five carbon atoms, have been suggested for use as refrigerants. U.S. Patent No. 4,788,352 - Smutny is directed to production of fluorinated C₅ to C₈ compounds having at least some degree of unsaturation. The Smutny patent identifies such higher olefins as being known to have utility as refrigerants, pesticides, dielectric fluids, heat transfer fluids, solvents, and intermediates in various chemical reactions. (See column 1, lines 11 - 22).

While the fluorinated olefins described in Smutny may have some level of effectiveness in heat transfer applications, it is believed that such compounds may also have certain disadvantages. For example, some of these compounds may tend to attack substrates, particularly general-purpose plastics such as acrylic resins and ABS resins. Furthermore, the higher olefinic compounds described in Smutny may also be undesirable in certain applications because of the potential level of toxicity of such compounds which may arise as a result of pesticide activity noted in Smutny. Also, such compounds may have a boiling point which is too high to make them useful as a refrigerant in certain applications.

Bromofluoromethane and bromochlorofluoromethane derivatives, particularly bromotrifluoromethane (Halon 1301) and bromochlorodifluoromethane (Halon 1211) have gained widespread use as fire extinguishing agents in enclosed areas such as airplane cabins and computer rooms. However, the use of various halons is being phased out due to their high ozone depletion. Moreover, as halons are frequently used in areas where humans are present, suitable replacements must also be safe to humans at concentrations necessary to suppress or extinguish fire.

JP 4 110388 A describes a heat transfer fluid for heat pumps and heat engines, comprising an organic compound represented by the molecular formula C₃HₘFₙ, where m is from 1 to 5, n is from 1 to 5 and m+n=6 and which has one double bond in the molecular structure.

RU 2 073 058 C1 describes an ozone-safe working mixture for refrigeration systems that contains tetrafluoroethane, an unsaturated fluorinated propyl hydrocarbon having the formula C₃FₙH₍₆₋ₙ₎, where n is from 1 to 6, and a hydrocarbon.

Applicants have thus come to appreciate a need for compositions that are potentially useful in vapor compression heating and cooling systems and methods, while avoiding one or more of the disadvantages noted above.

### SUMMARY

Applicants have found that the above-noted need, and other needs, can be satisfied by the use as a refrigerant composition, of a composition comprising 1,3,3,3-tetrafluoropropene (HFO-1234ze) or 2,3,3,3-tetrafluoropropene (HFO-1234yf), and a lubricant selected from polyol esters.

### DETAILED DESCRIPTION OF PREFERRED EMBODIMENTS

### THE COMPOSITIONS

The compositions described herein may include compounds of Formula II below: 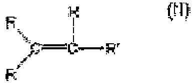 where each R is independently Cl, F, Br, I or H
R' is (CR₂)ₙY,
Y is CRF₂
and n is 0 or 1.
In highly preferred examples, Y is CF₃, n is 0 and at least one of the remaining Rs is F.

The compounds of the above identified Formula II are generally effective and exhibit utility in refrigerant compositions. However, applicants have surprisingly and unexpectedly found that certain of the compounds having a structure in accordance with the formulas described above exhibit a highly desirable low level of toxicity compared to other of such compounds. As can be readily appreciated, this discovery is of potentially enormous advantage and benefit for the formulation of refrigerant compositions. More particularly, applicants believe that a relatively low toxicity level is associated with compounds of Formula II, preferably wherein Y is CF₃, wherein at least one R on the unsaturated terminal carbon is H, and at least one of the remaining Rs is F. Applicants believe also that all structural, geometric and stereoisomers of such compounds are effective and of beneficially low toxicity.

In highly preferred examples, especially those which comprise the low toxicity compounds described above, n is zero. Thus, the compositions described herein may comprise one or more compounds selected from the group consisting of tetrafluoropropenes (HFO-1234), pentafluoropropenes (HFO-1225) and combinations of these.

The compounds for use in the present invention are 1, 3, 3, 3-tetrafluoropropene (HFO-1234ze); 2, 3, 3, 3-tetrafluoropropene (HFO-1234yf), and any and all stereoisomers of each of these. The applicant has discovered that such compounds have a very low acute toxicity level, as measured by inhalation exposure to mice and rats. On the other hand, applicants have found that a relatively high degree of toxicity may be associated with certain compounds, namely, those compounds which have more than one F on the terminal unsaturated carbon, or which do not have at least one H on the terminal unsaturated carbon. For example, applicants have discovered that 1,1,3,3,3-pentafluoropropene (HFO-1225zc) exhibits an unacceptably high degree of toxicity, as measured by inhalation exposure to mice and rats.

HFO-1225 and HFO-1234 are known materials and are listed in Chemical Abstracts databases. HFO-1225 is commercially available, from example from Syntex Chemical Co. Furthermore, methods are described generally in the patent literature for producing fluoroalkenes. For example, the production of fluoropropenes such as CF₃CH=CH₂ by catalytic vapor phase fluorination of various saturated and unsaturated halogen-containing C₃ compounds is described in U.S. Patent Nos. 2,889,379; 4,798,818 and 4,465,786. U.S. Patent No. 5,532,419 discloses a vapor phase catalytic process for the preparation of fluoroalkene using a chloro- or bromo- halofluorocarbon and HF. EP 974,571 discloses the preparation of 1,1,1,3-tetrafluoropropene by contacting 1,1,1,3,3- pentafluoropropane (HFC-245fa) in the vapor phase with a chromium-based catalyst at elevated temperature, or in the liquid phase with an alcoholic solution of KOH, NaOH, Ca(OH)₂ or Mg(OH)₂. In addition, methods for producing compounds disclosed herein are described generally in connection with concurrently filed United States Patent Application entitled "Process for Producing Fluorpropenes" bearing attorney docket number (H0003789 (26267)).

The compositions disclosed herein are believed to possess properties that are advantageous for a number of important reasons. For example, applicants believe, based at least in part on mathematical modeling, that the fluoroolefins for use in the present invention will not have a substantial negative affect on atmospheric chemistry, being negligible contributors to ozone depletion in comparison to some other halogenated species. The preferred compositions for use in the present invention thus have the advantage of not contributing substantially to ozone depletion. The preferred compositions for use in the present invention also do not contribute substantially to global warming compared to many of the hydrofluoroalkanes presently in use.

Preferably, the compositions for use in the present invention have a Global Warming Potential (GWP) of not greater than 150, more preferably not greater than 100 and even more preferably not greater than 75. As used herein, "GWP" is measured relative to that of carbon dioxide and over a 100 year time horizon, as defined in "The Scientific Assessment of Ozone Depletion, 2002, a report of the World Meteorological Association's Global Ozone Research and Monitoring Project,".

The compositions for use in the present invention also preferably have an Ozone Depletion Potential (ODP) of not greater than 0.05, more preferably not greater than 0.02 and even more preferably about zero. As used herein, "ODP" is as defined in "The Scientific Assessment of Ozone Depletion, 2002, A report of the World Meteorological Association's Global Ozone Research and Monitoring Project,".

### HEAT TRANSFER COMPOSITIONS

Although it is contemplated that the compositions for use in the present invention may include HFO-1234ze or HFO-1234yf in widely ranging amounts, it is generally preferred that refrigerant compositions for use in the present invention comprise HFO-1234ze or HFO-1234yf, in an amount that is at least about 50% by weight, and even more preferably at least about 70 % by weight, of the composition.

The compositions for use in the present invention may include other components for the purpose of enhancing or providing certain functionality to the composition, or in some cases to reduce the cost of the composition. For example, refrigerant compositions for use in the present invention, especially those used in vapor compression systems, include a lubricant, generally in amounts of from about 30 to about 50 percent by weight of the composition. Furthermore, the compositions for use in the present invention may also include a compatibilzer, such as propane, for the purpose of aiding compatibility and/or solubility of the lubricant. Such compatibilizers, including propane, butanes and pentanes, are preferably present in amounts of from about 0.5 to about 5 percent by weight of the composition. Combinations of surfactants and solubilizing agents may also be added to the compositions for use in the present invention to aid oil solubility, as disclosed by U.S. Patent No. 6,516,837. Commonly used refrigeration lubricants such as Polyol Esters (POEs) that are used in refrigeration machinery with hydrofluorocarbon (HFC) refrigerants are used with the refrigerant compositions for use in the present invention.

### METHODS AND SYSTEMS

The compositions for use in the present invention are useful in connection with numerous methods and systems, including as heat transfer fluids in methods and systems for transferring heat, such as refrigerants used in refrigeration, air conditioning and heat pump systems.

### HEAT TRANSFER METHODS

The preferred heat transfer methods disclosed herein generally comprise providing a composition for use in the present invention and causing heat to be transferred to or from the composition changing the phase of the composition. For example, the methods described herein provide cooling by absorbing heat from a fluid or article, preferably by evaporating the refrigerant composition for use in the present invention in the vicinity of the body or fluid to be cooled to produce vapor comprising the composition. Preferably the methods described herein include the further step of compressing the refrigerant vapor, usually with a compressor or similar equipment to produce vapor of the composition for use in the present invention at a relatively elevated pressure. Generally, the step of compressing the vapor results in the addition of heat to the vapor, thus causing an increase in the temperature of the relatively high pressure vapor. Preferably, the methods described herein include removing from this relatively high temperature, high pressure vapor at least a portion of the heat added by the evaporation and compression steps. The heat removal step preferably includes condensing the high temperature, high pressure vapor while the vapor is in a relatively high pressure condition to produce a relatively high pressure liquid comprising a composition for use in the present invention. This relatively high pressure liquid preferably then undergoes a nominally isoenthalpic reduction in pressure to produce a relatively low temperature, low pressure liquid. In such embodiments described herein, it is this reduced temperature refrigerant liquid which is then vaporized by heat transferred from the body or fluid to be cooled.

In another process embodiment of the disclosure, the compositions for use in the invention may be used in a method for producing heating which comprises condensing a refrigerant comprising the compositions for use in the present invention in the vicinity of a liquid or body to be heated. Such methods, as mentioned hereinbefore, frequently are reverse cycles to the refrigeration cycle described above.

### REFERENCE EXAMPLE 1

The coefficient of performance (COP) is a universally accepted measure of refrigerant performance, especially useful in representing the relative thermodynamic efficiency of a refrigerant in a specific heating or cooling cycle involving evaporation or condensation of the refrigerant. In refrigeration engineering, this term expresses the ratio of useful refrigeration to the energy applied by the compressor in compressing the vapor. The capacity of a refrigerant represents the amount of cooling or heating it provides and provides some measure of the capability of a compressor to pump quantities of heat for a given volumetric flow rate of refrigerant. In other words, given a specific compressor, a refrigerant with a higher capacity will deliver more cooling or heating power. One means for estimating COP of a refrigerant at specific operating conditions is from the thermodynamic properties of the refrigerant using standard refrigeration cycle analysis techniques (see for example, R.C. Downing, FLUOROCARBON REFRIGERANTS HANDBOOK, Chapter 3, Prentice-Hall, 1988).

A refrigeration /air conditioning cycle system is provided where the condenser temperature is about 66°C (150°F) and the evaporator temperature is about -37°C (-35°F) under nominally isentropic compression with a compressor inlet temperature of about 10°C (50°F). COP is determined for several compositions of the present invention over a range of 5 condenser and evaporator temperatures and reported in Table I below, based upon HFC-134a having a COP value of 1.00, a capacity value of 1.00 and a discharge temperature of 79°C (175 °F).

**TABLE I**

| REFRIGERANT COMPOSTION | Relative COP | Relative CAPACITY | DISCHARGE TEMPERATURE °C (°F) |
|---|---|---|---|
| HFO 1225ye | 1.02 | 0.76 | 70 (158) |
| HFO trans-1234ze | 1.04 | 0.70 | 74 (165) |
| HFO cis-1234ze | 1.13 | 0.36 | 68 (155) |
| HFO 1234yf | 0.98 | 1.10 | 76 (168) |

This example shows that certain of the preferred compounds for use with the present compositions each have a better energy efficiency than HFC-134a (1.02, 1.04 and 1.13 compared to 1.00) and the compressor using the present refrigerant compositions will produce discharge temperatures (70°C (158°F), 74°C (165°F) and 68°C (155°F) compared to 79°C (175°F)), which is advantageous since such result will likely lead to reduced maintenance problems.

### EXAMPLE 2

The miscibility of HFO-1225ye and HFO-1234ze with various refrigeration lubricants is tested. The lubricants tested are mineral oil (C3), alkyl benzene (Zerol 150), ester oil (Mobil EAL 22 cc and Solest 120), polyalkylene glycol (PAG) oil (Goodwrench Refrigeration Oil for 134a systems), and a poly(alpha-olefin) oil (CP6005-100). For each refrigerant/oil combination, three compositions are tested, namely 5, 20 and 50 weight percent of lubricant, with the balance of each being the compound of the present invention being tested.

The lubricant compositions are placed in heavy-walled glass tubes. The tubes are evacuated, the refrigerant compound in accordance with the present invention is added, and the tubes are then sealed. The tubes are then put into an air bath environmental chamber, the temperature of which is varied from about -50°C to 70°C. At roughly 10°C intervals, visual observations of the tube contents are made for the existence of one or more liquid phases. In a case where more than one liquid phase is observed, the mixture is reported to be immiscible. In a case where there is only one liquid phase observed, the mixture is reported to be miscible. In those cases where two liquid phases were observed, but with one of the liquid phases occupying only a very small volume, the mixture is reported to be partially miscible.

The polyalkylene glycol and ester oil lubricants were judged to be miscible in all tested proportions over the entire temperature range, except that for the HFO-1225ye mixtures with polyalkylene glycol, the refrigerant mixture was found to be immiscible over the temperature range of -50°C to -30°C and to be partially miscible over from - 20°C to 50°C. At 50 weight percent concentration of the PAG in refrigerant and at 60°C, the refrigerant/PAG mixture was miscible. At 70°C, it was miscible from 5 weight percent lubricant in refrigerant to 50 weight percent lubricant in refrigerant.

### REFERENCE EXAMPLE 3

The compatibility of the refrigerant compounds and compositions of the present invention with PAG lubricating oils while in contact with metals used in refrigeration and air conditioning systems is tested at 350° C, representing conditions much more severe than are found in many refrigeration and air conditioning applications.

Aluminum, copper and steel coupons are added to heavy walled glass tubes. Two grams of oil are added to the tubes. The tubes are then evacuated and one gram of refrigerant is added. The tubes are put into an oven at 350°F for one week and visual observations are made. At the end of the exposure period, the tubes are removed.

This procedure was done for the following combinations of oil and the compound of the present invention:
a) HFC-1234ze and GM Goodwrench PAG oil
b) HFC-1243zf and GM Goodwrench oil PAG oil
c) HFC-1234ze and MOPAR-56 PAG oil
d) HFC-1243zf and MOPAR-56 PAG oil
e) HFC-1225ye and MOPAR-56 PAG oil.

In all cases, there is minimal change in the appearance of the contents of the tube. This indicates that the refrigerant compounds and compositions of the present invention are stable in contact with aluminum, steel and copper found in refrigeration and air conditioning systems, and the types of lubricating oils that are likely to be included in such compositions or used with such compositions in these types of systems.

### COMPARATIVE EXAMPLE

Aluminum, copper and steel coupons are added to a heavy walled glass tube with mineral oil and CFC-12 and heated for one week at 350°C, as in Example 3. At the end of the exposure period, the tube is removed and visual observations are made. The liquid contents are observed to turn black, indicating there is severe decomposition of the contents of the tube.

CFC-12 and mineral oil have heretofore been the combination of choice in many refrigerant systems and methods. Thus, the refrigerant compounds and compositions of the present invention possess significantly better stability with many commonly used lubricating oils than the widely-used prior art refrigerant-lubricating oil combination.

## Claims

1. Use as a refrigerant composition, of a composition comprising 1,3,3,3-tetrafluoropropene (HFO-1234ze) or 2,3,3,3-tetrafluoropropene (HFO-1234yf), and a lubricant selected from polyol esters.

2. The use according to claim 1, wherein the HFO-1234ze or HFO-1234yf is present in an amount of at least 50 wt% of the refrigerant composition.

3. The use according to claim 2, wherein the HFO-1234ze or HFO-1234yf is present in an amount of at least 70 wt% of the refrigerant composition.

4. The use according to claim 1, wherein the lubricant is present in an amount of from 30 to 50 wt% of the refrigerant composition.

5. The use according to any preceding claim, wherein the refrigerant composition has as Global Warming Potential (GWP) of not greater than 150, more preferably not greater than 100 and even more preferably not greater than 75.

6. The use according to any preceding claim, in a refrigeration, air conditioning, or heat pump system.

## Patentansprüche

1. Verwendung einer Zusammensetzung, die 1,3,3,3-Tetrafluorpropen (HFO-1234ze) oder 2,3,3,3-Tetrafluorpropen (HFO-1234yf) und ein Schmiermittel, das aus Polyolestern ausgewählt ist, umfasst, als Kältemittelzusammensetzung.

2. Verwendung nach Anspruch 1, wobei das HFO-1234ze oder HFO-1234yf in einer Menge von mindestens 50 Gew.-% der Kältemittelzusammensetzung vorliegt.

3. Verwendung nach Anspruch 2, wobei das HFO-1234ze oder HFO-1234yf in einer Menge von mindestens 70 Gew.-% der Kältemittelzusammensetzung vorliegt.

4. Verwendung nach Anspruch 1, wobei das Schmiermittel in einer Menge von 30 bis 50 Gew.-% der Kältemittelzusammensetzung vorliegt.

5. Verwendung nach einem der vorhergehenden Ansprüche, wobei die Kältemittelzusammensetzung ein Treibhauspotential (Global Warming Potential, GWP) von nicht mehr als 150, weiter bevorzugt nicht mehr als 100 und noch weiter bevorzugt nicht mehr als 75 aufweist.

6. Verwendung nach einem der vorhergehenden Ansprüche in einem Kälteerzeugungs-, Klimatisierungs- oder Wärmepumpen-system.

## Revendications

1. Utilisation en tant que composition réfrigérante, d'une composition comprenant du 1,3,3,3-tétrafluoropropène (HFO-1234ze) ou du 2,3,3,3-tétrafluoropropène (HFO-1234yf), et d'un lubrifiant choisi parmi des esters de polyol.

2. Utilisation selon la revendication 1, le HFO-1234ze ou le HFO-1234yf étant présent en une quantité d'au moins 50 % en poids de la composition réfrigérante.

3. Utilisation selon la revendication 2, le HFO-1234ze ou le HFO-1234yf étant présent en une quantité d'au moins 70 % en poids de la composition réfrigérante.

4. Utilisation selon la revendication 1, le lubrifiant étant présent en une quantité de 30 à 50 % en poids de la composition réfrigérante.

5. Utilisation selon une quelconque revendication précédente, la composition réfrigérante possédant un potentiel de réchauffement global (PRG) de pas plus de 150, plus préférablement pas plus de 100 et encore plus préférablement pas plus de 75.

6. Utilisation selon une quelconque revendication précédente, dans un système de réfrigération, de conditionnement de l'air, ou de pompe à chaleur.
